Europäisches Patentamt

European Patent Office

Office européen des brevets

(19)

(11) Publication number: **0 164 843 B1**

# (12) EUROPEAN PATENT SPECIFICATION

(45) Date of publication of patent specification: **19.06.91**　(51) Int. Cl.⁵: **A61K 43/00**

(21) Application number: **85302634.2**

(22) Date of filing: **15.04.85**

(54) Organic amine phosphonic acid complexes for the treatment of calcific tumors.

(30) Priority: **04.06.84 US 616985**

(43) Date of publication of application:
**18.12.85 Bulletin 85/51**

(45) Publication of the grant of the patent:
**19.06.91 Bulletin 91/25**

(84) Designated Contracting States:
**BE CH DE FR GB IT LI NL SE**

(56) References cited:
**CA-A- 1 078 731**
**US-A- 4 104 366**
**US-A- 4 504 463**
**US-A- 4 508 704**

**ULLMANS ENCYKLOPAEDIE DER TECH-
NISCHEN CHEMIE, 4th edition, vol. 20, 1981;
VERLAG CHEMIE, Weinheim, Deerfield
Beach, Florida, Basel, pages 78-90**

(73) Proprietor: **THE DOW CHEMICAL COMPANY
2030 Dow Center Abbott Road P.O. Box 1967
Midland Michigan 48640-1967(US)**

(72) Inventor: **Simon, Jaime
Route 1, Box 120-G
Angleton, TX 77515(US)**
Inventor: **Volkert, Wynn A.
2203 Garden Drive
Columbia, MO 65212(US)**
Inventor: **Wilson, David A.
229 San Saba
Richwood, TX 77531(US)**
Inventor: **Troutner, David E.
402 Edgewood Avenue
Columbia, MO 65201(US)**
Inventor: **Goeckeler, William F.
617 Hemlock
Midland, MI 48640(US)**

(74) Representative: **Raynor, John et al
W.H. Beck, Greener & Co 7 Stone Buildings
Lincoln's Inn
London WC2A 3SZ(GB)**

EP 0 164 843 B1

## Description

Aminophosphonic acids are known to chelate metal ions. Particularly stable chelates are formed with metals from the alkaline earth and transition metal series.

The development of a bone metastasis is a common and often catastrophic event for a cancer patient. The pain, pathological fractures, frequent neurological deficits and forced immobility caused by these metastatic lesions significantly decreases the quality of life for the cancer patient. The number of patients that contract metastatic disease is large since nearly 50% of all patients who contract breast, lung or prostate carcinoma will eventually develop bone metastases. This indicates the prevalence of the disease. Bone metastases are also seen in patients with carcinoma of the kidney, thyroid, bladder, cervix and other tumors, but collectively, these represent less than 20% of patients who develop bone metastases. Metastatic bone cancer is rarely life threatening and occasionally patients live for years following the discovery of the bone lesions. Initially, treatment goals center on relieving pain, reducing requirements for narcotic medication and increasing ambulation. Clearly, it is hoped that some of the cancers can be cured.

The use of radionuclides for treatment of cancer metastatic to the bone dates back to the early 1950's. It has been proposed to inject a radioactive particle-emitting nuclide in a suitable form for the treatment of calcific lesions. It is desirable that such nuclides be concentrated in the fast growing portion of the bone with minimal amounts reaching the soft tissue and normal bone. Radioactive phosphorus compounds (P-32 and P-33) have been proposed. However, the nuclear and biolocalization properties limit the use of these compounds. (Kaplan, E., et al, Journal of Nuclear Medicine, Vol. 1, No. 1, page 1, 1960); (U.S. Patent 3,965,254).

Another attempt has been made using phosphorus compounds containing a boron residue. The compounds were injected into the body (intravenously) and accumulated in the skeletal system. The patient was then irradiated with neutrons in order to activate the boron and give a therapeutic radiation dose. (U.S. Patent 4,399,817).

In the above-mentioned procedures, it is not possible to give therapeutic doses to the tumor without substantial damage to normal tissues. In many cases, especially for metastic bone lesions, the tumor has spread throughout the skeletal system and amputation or irradiation of a portion of the body is not practical. (Seminars in Nuclear Medicine, Vol. IX, No. 2, April, 1979).

The use of diphosphonate complexed with Re-186 has also been proposed. (Mathieu, L. et al, Int. J. Applied Rad. & Isotopes, Vol. 30, pp. 725-727, 1979; Weinenger, J., Ketring, A. R., et al, Journal of Nuclear Medicine, Vol. 24, No. 5, page 125, 1983). However, the preparation and purification needed for this complex limits its utility and wide application.

Strontium-89 has also been proposed for patients with metastic bone lesions. However, the long half-life (50.4 days), high blood levels and low lesion to normal bone ratios limit the utility. (Firusian, N., Mellin, P., Schmidt, C. G., The Journal of Urology, Vol. 116, page 764, 1976; Schmidt, C. G., Firusian, N., Int. J. Clin. Pharmacol., 93:199-205, 1974).

A palliative treatment of bone metastases has been reported which employed I-131 labelled α-amino-(3-iodo-4-hydroxybenzylidene)diphosphonate (Eisenhut, M., Journal of Nuclear Medicine, Vol. 25, No. 12, pp. 1356-1361, 1984). The use of radioiodine as a therapeutic radionuclide is less than desirable due to the well known tendency of iodide to localize in the thyroid. Eisenhut lists iodide as one of the possible metabolites of this compound. In addition, any I-131 left over from the iodination reaction and not separated in the washing procedure also constitutes a threat to the thyroid. Indeed Eisenhut finds 0.1% of the injected dose present in the thyroid 24 hours after injection. The high energy of the gamma ray emission from I-131 leads to inferior quality images.

Canadian patent CA-A-1 078 731 describes an injectable solution incorporating Tc-99m and freeze-dried triethylene tetramine hexa (methylene phosphonic acid) for skeletal imaging. The Journal of Nuclear Medicine, June 1975, page 540, discloses several Tc-99m labelled polyfunctional phosphonates as skeletal imaging agents, i.e. ethylenediamine tetra(methylene phosphonic acid), triethylenediamine tetra (methylene phosphonic acid), diethylenetriamine penta (methylene phosphonic acid) and nitrilotri (methylene phosphonic acid)

It should also be recognized that the properties of the particular isotope are important. The disadvantage of any one property may be overcome by the superiority of one or more of the properties of either ligand or isotope and their combination as the complex must be considered in toto.

The following is a discussion of the criteria which must be considered in choosing any particular combination of radioisotope and ligand.

Firstly, the complex must be taken up preferentially by the bone rather than by soft tissue. Most particularly, uptake in neither liver nor bone marrow is desired. Uptake by muscle tissue, while not as damaging, is also undesirable, resulting in "inferior" imaging properties as well as unwanted dosage to non-target organs.

Another important criterion is the ratio of the amount of complex taken up by the cancerous bone to that by normal bone. High ratios are preferred since it is desired to treat the cancerous bone while irradiating the normal bone as little as possible.

The complex should be cleared from the blood rapidly for obvious reasons.

With respect to the complexed radionuclide, considerations of the nuclear properties are essential. First, the half-life must be sufficiently long to allow for localization in the bone without delivering excessive doses to non-target organs. When the complex is able to biolocalize rapidly, isotopes having shorter half-lives are useful. Isotopes with shorter half-lives provide an advantage since this permits the total dose to be given fractionally, allowing any non-target organs damaged by the radiation to recover between doses.

While for successful treatment of tumors the isotope must have sufficient particle emission, it is desirable, in order to quantify the localization of the complex, that the isotope also have sufficient gamma ray production that imaging is possible. The preferred gamma ray energy should be in the 100 to 200 keV $(16 \times 10^{-15}$ to $32 \times 10^{-15}$ joule) range although some isotopes having higher energies are potentially useful. The amount of gamma radiation should be sufficient to image, but not so great that the patient needs to be isolated to prevent him from becoming a source of radiation exposure to persons near him.

There is a need, therefore, for a system possessing the above criteria by which it is possible to deliver therapeutic radiation doses to calcific tumors with minimal doses to soft tissue or normal bone.

Such a system has now been found wherein specific radioactive metal ions are complexed to a phosphorus-containing ligand. Certain of these complexes have been shown to be very selective for the skeletal system with very low soft tissue uptake. The material not taken up by bone is efficiently cleared through the kidneys into the bladder. The complexes also tend to concentrate in areas of fast-growing bone much more readily than in normal bone. The radionuclides used are particle-emitting and a high radiation dose is delivered in the area where they are deposited. Thus, therapeutic radiation doses can be delivered specifically to calcific tumors.

The invention provides a therapeutically effective complex of a paticle-emitting radionuclide which is gadolinium-159 (Gd-159), holmium-166 (Ho-166), lutetium-177 (Lu-177), samarium-153 (Sm-153) or ytterbium-175 (Yb-175) with an aminophosphonic acid derivative which is ethylenediaminetetramethylenephosphonic acid (EDTMP), diethylenetriaminepentamethylenephosphonic acid (DTPMP), hydroxyethylethylenediaminetrimethylenephosphonic acid (HEEDTMP), nitrilotrimethylenephosphonic acid (NTMP) or tris(2-aminoethyl) aminehexamethylenephosphonic acid (TTHMP) or a physiologically acceptable salt of such a complex.

The proposed use for the complexes of this invention is the therapeutic treatment of calcific tumors. These include primary tumors, where the skeletal system is the first site of involvement, and metastic bone cancer where the neoplasm spreads from other primary sites, e.g. prostate, breast, into the skeletal system. This invention provides a method of alleviating pain by delivering a therapeutic radiation dose to the aforementioned calcific tumors. This invention also provides a means of reducing the size of or destroying the calcific tumors by delivering a therapeutic radiation dose.

The amino phosphonic acid derivatives can be prepared by a number of known synthetic techniques. Of particular importance is the reaction of the appropriate amine with a carbonyl compound (aldehyde or ketone) and phosphorous acid or derivative thereof.

Methods which give alkyl phosphonic and hydroxyalkyl substituents on the amine nitrogens are well known (U.S. 3,398,198).

Compounds with which the radionuclides may be complexed are ethylenediaminetetramethylenephosphonic acid (EDTMP), diethylenetriaminepentamethylenephosphonic acid (DTPMP), hydroxyethylethylenediaminetrimethylenephosphonic acid (HEEDTMP), nitrilotrimethylenephosphonic acid (NTMP) and tris(2-aminoethyl)aminehexamethylenephosphonic acid (TTHMP).

The particle-emitting nuclides used in the practice of the invention are Sm-153, Yb-175, Lu-177 and Gd-159.

While Sm-153, Yb-175, Lu-177 and Gd-159 have been exemplified as the rare earth radionuclides employed with the complexing agents above, H0-166 may alternatively be employed.

For the purpose of convenience the abbreviations given in the parentheses above will be used to denote the respective radionuclides and aminophosphonic acid derivatives hereinafter.

A more preferred embodiment of the present invention is a therapeutically effective complex of a particle-emitting radionuclide selected from the group consisting of Gd-159, Lu-177, Sm-153, and Yb-175 with an aminophosphonic acid derivative selected from the group consisting of EDTMP, DTPMP, HEEDTMP, NTMP and TTHMP.

A particularly preferred embodiment of the present invention is a therapeutically effective complex of the particle-emitting radionuclide selected from the group consisting of Lu-177, Sm-153 and Yb-175 with an aminophosphonic acid derivative selected from the group consisting of EDTMP, DTPMP, HEEDTMP, NTMP and TTHMP.

The most preferred embodiment of the present invention is a therapeutically effective complex of Sm-153 with an aminophosphonic acid derivative selected from the group consisting of EDTMP, DTPMP, HEEDTMP, and TTHMP.

For the purpose of the present invention, therapeutically effective complexes described herein and physiologically-acceptable salts thereof are considered equivalent. Physiologically-acceptable salts refer to the acid addition salts of those bases which will form a salt with at least one acid group of the complex and which will not cause an adverse physiological effect when administered to an animal at dosages consistent with good pharmacological activity. Suitable bases include, for example, the alkali metal and alkaline earth metal hydroxides, carbonates, and bicarbonates such as sodium hydroxide, potassium hydroxide, calcium hydroxide, potassium carbonate, sodium bicarbonate, magnesium carbonate and the like, ammonia, primary, secondary and tertiary amines and the like. Physiologically-acceptable salts of the present invention may be prepared by treating the complex having at least one acid group with an appropriate base.

Radionuclides can be produced in several ways. In a reactor, a nuclide is bombarded with neutrons to obtain a nuclide with additional neutrons in its nucleus.

e.g. Sm-152 + neutron → Sm-153 + gamma

Another method of obtaining radioisotopes is by bombarding nuclides with linear accelerator or cyclotron-produced particles. Yet another way of obtaining radioisotopes is to isolate them from fission product mixtures. The method of obtaining the radionuclide is not critical to the present invention.

When aqueous solutions of metal ions are mixed with solutions containing complexing agents, such as those described in this invention, a complex between the metal ion and the ligand can be formed as shown by the equation below.

$$M + L \rightleftharpoons M \cdot L$$

The reaction is believed to be an equilibrium such that the concentrations of metal (M) and complexing agent (L) can affect the concentration of species present in solution. Competing side reactions, such as metal hydroxide formation, can also occur in aqueous solution.

$$xM + yOH^- \rightarrow M_x(OH)_y$$

The $OH^-$ concentration in solution which is related to pH is, therefore, an important parameter to be considered. If the pH is too high, the metal tends to form metal hydroxides rather than complexes. The complexing agents may also be affected by low pH. Complexation may require the loss of protons; therefore at low pH, conditions may not be favorable for complexation to occur. The complexes of this invention can be formed at several pH's. Consideration must be given to the solubility characteristics of the ligand, radionuclide, and complex. Although complexation will occur at other pH values, pH from 5-11 is preferred for complexation.

The metal and ligand may be combined under any conditions which allow the two to form a complex. Generally, mixing in water at a controlled pH (the choice of pH is dependent upon the choice of ligand and metal) is all that is required. In some cases heating may be required to obtain maximum complex yield.

The ratio of ligand to metal is a result of two competing considerations. As indicated above the ligand and metal are believed to be in equilibrium with the complex. On the one hand, it is desirable to have a large quantity of ligand (L), so that there is a minimum amount of free metal (M), because the uncomplexed metal may cause serious side effects in the patient. On the other hand, excess free ligand may compete for sites in the target, thus rendering the treatment less effective. The fact that the ligands within the scope of the invention are capable of complexing with varying molar ratios of metal adds a degree of complexity to these considerations. Although it is difficult to generalize, it is believed that the ligand to metal molar ratio is desirably 0.1:1 to 3000:1, preferably 1:1 to 2000:1, more preferably 1:1 to 1000:1.

The samarium used in the following example was either natural $Sm_2O_3$ (99.9 percent from Spex Industries) or isotopically enriched (99.06 percent Sm-152) $Sm_2O_3$.

The Sm-153 used in this study was produced by neutron irradiation at the University of Missouri Research Reactor. Preliminary studies were carried out using Sm-153 produced by short (5-30 minutes) irradiations of natural $Sm_2O_3$, in the reactor's pneumatic tube system. The specific activity of Sm-153 produced by this method was 0.5-3.0 Ci/g (18.5 to 111 GBq/g).

The majority of this work was carried out using Sm-153 produced by irradiating 99.06 percent enriched $^{152}Sm_2O_3$ in the first row reflector at a neutron flux of $1 \times 10^{14}$ neutron/$cm^2 \cdot sec$. Irradiations were generally carried out for 50-60 hours, yielding a Sm-153 specific activity of 1000 - 1300 Ci/g ($37 \times 10^3$ to $48.1 \times 10^3$ GBq/g).

To irradiate $Sm_2O_3$ for production of Sm-153, the desired amount of target was first weighed into a quartz vial, the vial flame sealed under vacuum and welded into an aluminum can. The can was irradiated for the desired length of time, cooled for several hours and opened remotely in a hot cell. The quartz vial was removed and transferred to a glove box, crushed into a glass vial which was then sealed with a rubber septum and an aluminum crimp cap. One milliliter of 1-4 M HCl was then added to the vial via syringe to dissolve the $Sm_2O_3$. Once dissolved, the solution was diluted to the appropriate volume by addition of water. The solution was removed from the original dissolution vial which contains the chards of the crushed quartz vial, and transferred via syringe to a clean glass serum vial. This solution was then used for complex preparation. Similar procedures were used to prepare other radionuclides, e.g. Lu-177, Yb-175, Gd-159.

The various complexes employed in this invention were prepared as follows: the desired amount of ligand was placed in a vial and dissolved by addition of water. At some higher ligand concentrations, it was necessary to add base in order to completely dissolve the ligand. Heating was also found to be useful for dissolving the ligands. The appropriate amount of the samarium or other radionuclides in the stock solution described above was then added to the ligand solution. The pH of the resulting solution was then raised to the appropriate level by addition of NaOH. The solution was heated to $60^°$-$70^°$C for 30 minutes in a water bath to insure maximum complex formation. The pH of the solution was then adjusted to 7-8 by addition of 1 M HCl.

The complex yield was determined by placing 5-20 microliters (depending on activity) of the complex solution onto a 0.5 $cm^3$ column of a commercially available synthetic organic cation exchange resin. The column was then eluted with two separate 10 milliliter volumes of isotonic saline. The anionic complexes are not retained by the resin and were eluted by the saline solution, while any uncomplexed metal was retained on the column. The eluant solutions and the column were then counted for the characteristic emission of the particular radionuclide, e.g. 103 keV ($16.5 \times 10^{-15}$ joule) gamma ray of Sm-153. The complex yield was obtained by adding the counts in the eluant solutions and dividing by the total of the eluant solutions and the column.

This invention provides a means of delivering a therapeutic radiation dose to calcific tumors. It may also be desirable in the cases where the radionuclide has imageable gamma photons to inject a "sub-therapeutic" dose and determine the fate of the radionuclide using a scintillation camera prior to injecting a therapeutic radiation dose. Therefore, the levels of radiation injected could be as low as 1 mCi (37 MBq) for imaging prior to the therapy. Therapeutic doses will be greater. The dose to the tumor may range from 100 to 10,000 rads (1 Gy to 100 Gy). The preferred dose to the tumor ranges from 1,000 to 8,000 rads (10 Gy to 80 Gy). For complexes such as Sm-153-EDTMP amounts ranging from 0.1 mCi/kg body weight to 3 mci/kg body weight ($0.4 \times 10^{10}$ to $11 \times 10^{10}$ Bq/kg) are preferred. The amount of activity required to deliver a therapeutic dose may vary with the individual radionuclides. Individual doses may be given in one injection or fractionated into several injections totaling the aforementioned dose per treatment.

Biodistribution of various complexes of the present invention was studied in rats and rabbits.

Studies to determine the qualitative dis-tribution of the various complexes of the present invention were conducted by injecting the complexes into rats and obtaining the gamma ray images of the entire animal at various times up to two hours after injection.

Male Spague Dawley rats were anesthetized by injection with sodium pentabarbitol and the jugular vein cannulated. The animals were then injected with 50 to 150 microliters of the various complexes and gamma ray images were taken on a Nuclear Chicago® Pho-Gamma® II scintillation camera immediately after injection and approximately every half hour thereafter for two hours.

Quantitative biodistributions were obtained by injecting 50-100 microliters of the complex solution into the tail vein of unanesthetized male Spague Dawley rats. The rats were then placed in cages lined with absorbent paper in order to collect all urine excreted prior to sacrifice. After two hours, the rats were sacrificed by cervical dislocation and the various tissues dissected out. The samples were then rinsed with saline, blotted dry on absorbent paper and weighed. The samples were counted with a Nal uptake counter

approximately one foot from the face of the crystal, in order to minimize geometry differences between the different size samples.

To minimize differences in age between groups of animals, all rats used were in the 160-220 gram weight range. All animals were kept "in-house" for at least one week prior to use to minimize the effects of stress that occur during shipping.

The various complexes of the present invention were also evaluated in rabbits. The animals used in these studies were male New Zealand white rabbits in the 2.6 to 3.2 kilogram weight range. The animals were kept "in-house" for at least a week prior to use.

The rabbits were injected with 100-250 microliters of the complex solution via a cannula placed in the marginal ear vein. In studies where blood clearance was measured, blood samples were taken through a heparinized cannula placed in the marginal vein of the ear not used for injection of the complex.

Three hours after injection, a blood sample was taken by cardiac puncture and the animal was then sacrificed by injection of a commercial euthanasia solution. After sacrifice, images were obtained by placing the carcass directly on the face of a large field of view scintillation camera.

Uptake of the complexes in the lesion compared to uptake in normal bone, or lesion/normal bone ratios, is a particularly important parameter for calculating the complex suitability as a therapeutic agent. To determine the lesion to normal bone ratios, a modified drill hole method (Subramanian, G. et al., 19th lit. Annual Meetings of S.N.M., Bern, Switzerland, September 8-11, 1981) was used.

In order to simulate uptake in rapidly growing bone, such as that found in cancerous bone, two holes were drilled into the surface of the tibia of a rabbit in order to damage the bone. Seven to ten days later, the animal was injected with the complex. After three hours, the animal was anesthetized and imaged using an Anger camera and pinhole collimator.

The following examples are illustrative of the present invention and are not to be construed as limiting the scope thereof.

## Example 1

Into a suitable reaction vessel equipped with a thermometer, magnetic stirring bar, dropping funnel, and an atmosphere of nitrogen were charged phosphorous acid (94.5 g) and degassed water (100 ml). Dissolution of the phosphorous acid was achieved by stirring and the solution was then treated with concentrated hydrochloric acid (112 ml). The dropping funnel was charged with ethylenediamine (15 g) and adjusted to allow dropwise addition of the diamine to the acidic solution. When addition was complete a heating mantle was installed and the solution refluxed for one hour. At the end of this time the dropping funnel was charged with formaldehyde (85 g of a 37% aqueous solution) which was added dropwise over a two-hour period with continued heating to maintain reflux during the addition. After all the formaldehyde was added, the reaction mixture was stirred under reflux for an additional two hours, then allowed to cool slowly overnight during which time the product precipitated. Vacuum filtration followed by cold water washing yielded ethylenediaminetetramethylenephosphonic acid (EDTMP).

## Example 2

25 to 35 milligrams of EDTMP prepared in Example 1 was weighed into a vial and dissolved using 0.75 ml of distilled water. To this, 0.25 ml of Sm-153 (~ 10 mCi(~ 370 MBq) in dilute HCl was added. The pH of the resulting solution was then adjusted to 10 by addition of NaOH. The resulting solution was heated to $60°C - 70°C$ for 30 minutes in a water bath to ensure maximum complex formation. The pH of the solution was then adjusted to 7-8 by addition of 1 M HCl. The yield of the complex was over 95 percent.

Laboratory rats were injected with the above complex (50-100 $\mu\ell$) via the tail vein. After 2 hours, the animals were sacrificed by cervical dislocation and organs and tissues removed. Samples were counted with a NaI uptake counter to determine the biolocalization of the complex. It was found that a significant amount (55-65 percent) of the activity was concentrated in the skeletal system with very little soft tissue uptake. Most of the activity not found in the skeleton was cleared through the kidneys into the bladder. Scintillation scans of animals treated in the same manner showed the activity concentrating in the skeletal system. The lesion to normal bone ratio (drill hole model) (This data was obtained by the method of Subramanian, G., McAfee, J. G., et al, 19th Int. Annual Meeting of S.N.M., Bern, Switzerland, September 8-11, 1981.) for this complex was approximately equal to that of Tc-99m-MDP (MDP refers to methylene diphosphonate), a commercially available diagnostic bone agent.

## Example 3

Into a suitable reaction vessel equipped with a thermometer, magnetic stirring bar, dropping funnel, and an atmosphere of nitrogen were charged phosphorous acid (94.5g) and degassed water (100 ml). Dissolution of the phosphorous acid was achieved by stirring and the solution was then treated with concentrated hydrochloric acid (112 ml). The dropping funnel was charged with diethylenetriamine (20.6 g) and adjusted to allow dropwise addition of the amine to the acidic solution. When addition was complete a heating mantle was installed and the solution refluxed for one hour. At the end of this time the dropping funnel was charged with formaldehyde (85 g of a 37% aqueous solution) which was added dropwise over a two-hour period with continued heating to maintain reflux during the addition. After all the formaldehyde was added, the reaction mixture was stirred under reflux for an additional two hours, then allowed to cool. Diethylenetriaminepentamethylenephosphonic acid (DTPMP) was isolated from the reaction mixture.

Example 4

20 to 30 milligrams of DTPMP prepared in Example 3 was weighed into a vial and dissolved using 0.75 ml of distilled water. To this, 0.25 ml of Sm-153 (~ 10 mCi(~ 370 MBq)) in dilute HCl was added. The pH of the resulting solution was then adjusted to 10 by addition of NaOH. The solution was then heated to 60° C-70° C for 30 minutes in a water bath to ensure maximum complex formation.

The pH of the solution was then adjusted to 7-8 by addition of 1 M HCl. The yield of the complex was over 95 percent.

The above complex was tested in rats. The results in rats showed ~ 30 percent of the activity in the skeletal system with very little activity in any soft tissue.

Example 5

Into a suitable reaction vessel equipped with a thermometer, magnetic stirring bar, dropping funnel, and an atmosphere of nitrogen were charged phosphorous acid (94.5 g) and degassed water (100 ml). Dissolution of the phosphorous acid was achieved by stirring and the solution was then treated with concentrated hydrochloric acid (112 ml). The dropping funnel was charged with N-hydroxyethylethylenediamine (34.6 g) and adjusted to allow dropwise addition of the diamine to the acidic solution. When addition was complete a heating mantle was installed and the solution refluxed for one hour. At the end of this time the dropping funnel was charged with formaldehyde (85 g of a 37% aqueous solution) which was added dropwise over a two hour period with continued heating to maintain reflux during the addition. After all the formaldehyde was added, the reaction mixture was stirred under reflux for an additional two hours, then allowed to cool. Hydroxyethylethylenediaminetrimethylenephosphonic acid (HEEDTMP) was isolated from the reaction mixture.

Example 6

30 to 40 milligrams of HEEDTMP prepared in Example 5 was weighed into a vial and dissolved using 0.75 ml of distilled water. To this, 0.25 ml of Sm-153 (~ 10 mCi(~ 370 MBq)) in dilute HCl was added. The pH of the resulting solution was then adjusted to 10 by addition of NaOH. The solution was then heated between 60° C and 70° C for 30 minutes in a water bath to ensure maximum complex formation. The pH of the solution was then adjusted to 7-8 by addition of 1 M HCl. The yield of the complex was over 95 percent.

Example 7

Into a suitable reaction vessel equipped with a thermometer, magnetic stirring bar, dropping funnel, and an atmosphere of nitrogen were charged phosphorous acid (57.7 g) and degassed water (50 ml). Dissolution of the phosphorous acid was achieved by stirring and the solution was then treated with concentrated hydrochloric acid (50 ml). The dropping funnel was charged with tris(2-aminoethyl)amine (13.7 g) and adjusted to allow dropwise addition of the amine to the acidic solution. When addition was complete a heating mantle was installed and the solution refluxed for one hour. At the end of this time the dropping funnel was charged with formaldehyde (51 g of a 37% aqueous solution) which was added dropwise over a two-hour period with continued heating to maintain reflux during the addition. After all the formaldehyde was added, the reaction mixture was stirred under reflux for an additional two hours, then allowed to cool. Tris(2-aminoethyl)aminehexamethylenephosphonic acid (TTHMP) was isolated from the reaction.

Example 8

48 to 53 milligrams of TTHMP prepared in Example 7 was weighed into a vial and dissolved using 0.75 ml of distilled water. To this solution, 0.25 ml of Sm-153 (~10 mCi(~ 370 MBq)) in dilute HCl was added. The pH of the resulting solution was adjusted to 10 by addition of NaOH. The solution was then heated between 60°C and 70°C for 30 minutes in a water bath to ensure maximum complex formation. The pH of the solution was then adjusted to 7-8 by addition of 1 M HCl.

The complexes of Examples 2, 4, 6 and 8 were tested in rats. The two-hour rat biodistribution data for these complexes is shown in Table I.

## TABLE I

| | Example Nos. | | | |
|---|---|---|---|---|
| | 2 | 4 | 6 | 8 |
| % Dose in Skeleton* | 58 ±4 | 30 ±10 | 57 ±7 | 28 ±4 |
| Blood | 0.032 ±0.016 | 0.16 ±0.12 | 0.035 ±0.006 | 0.25 ±0.14 |
| Liver | 0.25 ±0.04 | 0.27 ±0.13 | 0.45 ±0.07 | 0.18 ±0.02 |
| Urine | 49 ±4 | 74 ±8 | 50 ±4 | 65 ±9 |
| Bone/Blood | 1800 ±1200 | 224 ±150 | 1300 ±160 | 80 ±20 |
| Bone/Muscle | 1500 ±500 | 220 ±130 | 1300 ±180 | 410 ±75 |

Number of rats tested = 5

*Based on % dose in femur X 25

### Example 9

35 to 45 milligrams of EDTMP prepared according to Example 1 was weighed into a vial and dissolved using 0.75 ml of distilled water. To this solution, 0.25 ml of Yb-175 in dilute HCl was added. The pH of the resulting solution was adjusted to 10 by addition of NaOH. The solution was then heated between 60°C and 70°C for 30 minutes in a water bath to ensure maximum complex formation. The pH of the solution was then adjusted to 7-8 by addition of 1 M HCl.

### Example 10

55 to 60 milligrams of DTPMP prepared according to Example 3 was weighed into a vial and dissolved using 0.75 ml of distilled water. To this solution, 0.25 ml of Yb-175 in dilute HCl was added. The pH of the resulting solution was adjusted to 10 by addition of NaOH. The solution was then heated between 60°C and 70°C for 30 minutes in a water bath to ensure maximum complex formation. The pH of the solution was then adjusted to 7-8 by addition of 1 M HCl.

### Example 11

50 to 55 milligrams of HEEDTMP prepared according to Example 5 was weighed into a vial and dissolved with 0.75 ml of distilled water. To this solution, 0.25 ml of Yb-175 in dilute HCl was added. The pH of the resulting solution was adjusted to 10 by addition of NaOH. The solution was then heated between 60°C and 70°C for 30 minutes in a water bath to ensure maximum complex formation. The pH of the solution was then adjusted to 7-8 by addition of 1 M HCl.

Example 12

Into a suitable reaction vessel equipped with a thermometer, magnetic stirring bar, dropping funnel, and an atmosphere of nitrogen were charged phosphorous acid (94.5 g) and degassed water (100 ml). Dissolution of the phosphorous acid was achieved by stirring and the solution was then treated with concentrated hydro-chloric acid (112 ml). The dropping funnel was charged with ammonium chloride (17.2 g in an aqueous solution) and adjusted to allow dropwise addition of the ammonium chloride to the acidic solution. When addition was complete a heating-mantle was installed and the solution refluxed for one hour. At the end of this time the dropping funnel was charged with formaldehyde (85 g of a 37% aqueous solution) which was added dropwise over a two-hour period with continued heating to maintain reflux during the addition. After all the formaldehyde was added, the reaction mixture was stirred under reflux for an additional two hours, then allowed to cool, yielding nitrilotrimethylenephosphonic acid (NTMP).

Example 13

50 to 55 milligrams of NTMP prepared in Example 12 was weighed into a vial and dissolved with 0.75 ml of distilled water. To this solution, 0.25 ml of Yb-175 in dilute HCl was added. The pH of the resulting solution was then adjusted to 10 by addition of NaOH. The solution was then heated between 60°C and 70°C for 30 minutes to optimize complex formation. The pH of the solution was then adjusted to 7-8 by addition of 1 M HCl.

Complexes of Examples 9, 10, 11 and 13 were tested in rats. The biodistribution data are shown in Table II.

### TABLE II

| | Example Nos. | | | |
|---|---|---|---|---|
| | 9 | 10 | 11 | 13 |
| % Dose in Skeleton* | 50 ±7 | 25 ±2 | 56 ±2 | 63 ±5 |
| Blood | 0.074 ±0.027 | 0.116 ±0.040 | 0.231 ±0.044 | 0.204 ±0.059 |
| Liver | 0.138 ±0.036 | 0.112 ±0.027 | 0.214 ±0.082 | 0.236 ±0.072 |
| Bone/Blood | 562 ±130 | 179 ±60 | 190 ±40 | 256 ±90 |
| Bone/Muscle | 619 ±192 | 366 ±96 | 572 ±214 | 876 ±239 |

Number of rats tested = 5

*Based on % dose in femur X 25

Example 14

50 to 55 milligrams of EDTMP prepared in Example 1 was weighed into a vial and dissolved with 0.75 ml of distilled water. To this solution, 0.25 ml of Lu-177 in dilute HCl was added. The pH of the resulting solution was adjusted to 10 by addition of NaOH. The solution was then heated between 60°C and 70°C for 30 minutes to optimize complex formation. The pH of the solution was then adjusted to 7-8 by addition of 1 M HCl.

Example 15

55 to 60 milligrams of HEEDTMP prepared according to Example 5 was weighed into a vial and dissolved with 0.75 ml of distilled water. To this, 0.25 ml of Lu-177 in dilute HCl was added. The pH of the resulting solution was adjusted to 10 by addition of NaOH. The solution was then heated between 60°C and 70°C for 30 minutes to optimize complex formation. The pH of the solution was then adjusted to 7-8 by addition of 1 M HCl.

Complexes of Examples 14 and 15 were tested in rats. The biodistribution data are shown in Table III.

## TABLE III

| | Example Nos. | |
| --- | --- | --- |
| | 14 | 15 |
| % Dose in Skeleton* | 51 ±5 | 58 ±3 |
| Blood | 0.10 ±0.06 | 0.17 ±0.08 |
| Liver | 0.48 ±0.15 | 1.9 ±0.7 |
| Kidneys | 0.28 ±0.03 | 0.30 ±0.02 |
| Muscle | 0.44 ±0.27 | 0.56 ±0.16 |
| Bone/Blood | 500 ±220 | 370 ±320 |
| Bone/Muscle | 1380 ±1900 | 570 ±180 |

Number of rats tested = 5

* Based on % dose in femur X 25

Comparative Example

Two commercially available non-nitrogen-containing phosphonic acid compounds which are well known as diagnostically useful compounds when complexed with Tc-99m were complexed with Sm-153 and tested in the manner of the complexes of the present invention. The data comparable to that given for the examples of the invention is shown in Table A below:

## TABLE A.    Two Hour Biodistribution of Complexes in Rats

|  | $^{153}$Sm-MDP*** | $^{153}$Sm-HEDP* |
|---|---|---|
| % Dose in Skeleton** | 1.65 ±0.43 | 20.5 ±1.4 |
| Blood | 0.23 ±0.19 | 13.1 ±2.6 |
| Liver | 84.5 ±3.8 | 3.5 ±0.9 |
| Kidney | 0.45 ±0.19 | 0.99 ±0.44 |
| Urine | 0.20 ±0.14 | 41.3 ±4.6 |
| Bone/Blood | 8.6 ±5.0 | 1.3 ±0.3 |
| Bone/Muscle | 7.1 ±3.2 | 10.5 ±2.9 |

Number of rats tested = 5

\* 1-hydroxyethylidine-1,1-diphosphonic acid (HEDP)

\*\* Based on % dose in femur X 25

\*\*\*Methylenediphosphonate

It should be noted that the comparative examples in above Table A are of two non-nitrogen-containing phosphonic acid complexes. These have certain undesirable properties which make them inferior to the complexes of the invention. Thus, the Sm-MDP shows high liver uptake while the Sm-HEDP has both low skeletal uptake and poor clearance from the blood.

Example 16

The complex tested on rats in Example 2 (Sm-153-EDTMP) was also tested in rabbits in a similar manner. Results of biodistribution (averaged for 5 rabbits) are summarized in Table IV.

TABLE IV

| % Dose in Skeleton* | 66 ± 5 |
| --- | --- |
| Blood | 0.12 ± 0.10 |
| Liver | 0.95 ± 0.42 |
| Urine | 34 ± 4 |
| Bone/Blood | 900 ± 800 |
| Bone/Muscle | 1200 ± 400 |

* Based on % dose in femur X 20.1.

Example 17

An English Setter with a tumor in his pelvis was injected with ~19 mCi (700 MBq) of the complex of Example 2 (Sm-153-EDTMP). After 2 hours, the dog was imaged using a scintillation camera. The scintillation scan showed high uptake of the complex in the tumor and looked very similar to an earlier scan performed using Tc-99m-MDP. The uptake ratio of the Sm-153 complex in the tumor to normal bone was very similar to that of the Tc-99m complex. A scintillation scan of the dog five days after treatment gave similar results. Seven days after the treatment the dog appeared to be in less pain as evidenced by an increase in its activity.

Example 18

A series of rats was injected with the complex of Example 2 (Sm-153-EDTMP) and sacrificed at various intervals. The rat biodistribution data is summarized in Table V. This data shows rapid biolocalization properties, e.g. bone uptake and blood clearance, as well as permanent localization of the activity in the skeletal system.

Example 19

An Irish Setter with a tumor in the femur was injected with the complex of Example 2 (Sm-153-EDTMP). The leg was amputated and samples of the tumor and normal bone were counted for Sm-153. The amount of Sm-153 in the tumor was 15-20 times that of non-lesionous bone from the same leg.

Example 20

A series of 5 rabbits was injected with the complex of Example 2 (Sm-153-EDTMP). Less than 0.15 percent of the injected dose was found in the bone marrow.

## TABLE V

### BIODISTRIBUTION OF Sm-153-EDTMP OVER TIME IN RATS

| ORGAN | % Administered Dose After | | | | | | | |
|---|---|---|---|---|---|---|---|---|
| | 15 Min. | 30 Min. | 1 Hour | 2 Hours | 5 Hours | 24 Hours | 48 Hours | 72 Hours |
| Skeleton* | 48 ±4 | 53 ±2 | 58 ±3 | 58 ±4 | 59 ±4 | 52 ±3 | 60 ±1 | 57 ±4 |
| Blood | 5.852 ±.553 | 2.304 ±.476 | .532 ±.395 | .032 ±.016 | .008 ±.019 | .007 ±.002 | .006 ±.001 | .006 ±.001 |
| Liver | .959 ±.137 | .526 ±.108 | .322 ±.073 | .252 ±.038 | .370 ±.080 | .349 ±.021 | .458 ±.079 | .492 ±.115 |
| Kidneys | 1.745 ±.334 | .805 ±.069 | .466 ±.126 | .254 ±.035 | .364 ±.055 | .250 ±.087 | .286 ±.028 | .216 ±.036 |
| Urine | 28 ±3 | 42 ±5 | 47 ±3 | 49 ±4 | 46 ±3 | 55 ±2 | 50 ±2 | 53 ±1 |
| Bone/Muscle | 30 ±2 | 70 ±30 | 300 ±200 | 1500 ±500 | 3600 ±1900 | 2400 ±600 | 2800 ±400 | 3400 ±800 |
| Bone/Blood | 7 ±1 | 20 ±4 | 120 ±60 | 1800 ±1400 | 4200 ±3700 | 6700 ±1400 | 8200 ±1600 | 7800 ±1600 |

Number of rats tested = 5

*Based on % dose in femur x 25

Example 21

48 to 53 milligrams of EDTMP prepared according to Example 1 was weighed into a vial and dissolved with 0.75 ml of distilled water. To this solution, 0.25 ml of Gd-159 in dilute HCl was added. The pH of the resulting solution was adjusted to 10 by addition of NaOH. The solution was then heated between 60°C and 70°C in a water bath to optimize complex formation. The pH of the solution was then adjusted to 7-8 by addition of 1 M HCl.

Example 22

55 to 60 milligrams of HEEDTMP prepared according to Example 5 was weighed into a vial and dissolved with 0.75 ml of distilled water. To this solution, 0.25 ml of Gd-159 in dilute HCl was added. The pH of the resulting solution was adjusted to 10 by addition of NaOH. The solution was then heated between 60°C and 70°C in a water bath to optimize complex formation. The pH of the solution was then adjusted to 7-8 by addition of 1 M HCl.

Complexes of Examples 21 and 22 were tested in rats. The biodistribution data are shown in Table VI.

TABLE VI

|  | % Dose in Example No. | |
|---|---|---|
|  | 21 | 22 |
| Skeleton* | 57 ±3 | 60 ±4 |
| Blood | 0.15 ±0.03 | 0.14 ±0.02 |
| Liver | 0.25 ±0.04 | 0.57 ±0.07 |
| Kidneys | 0.33 ±0.09 | 0.58 ±0.11 |
| Muscle | 0.56 ±0.15 | 0.76 ±0.42 |
| Bone/Blood | 305 ±42 | 335 ±35 |
| Bone/Muscle | 577 ±192 | 548 ±419 |

*Calculated from % dose femur x 25

EP 0 164 843 B1

**Claims**

1. A therapeutically effective complex of a paticle-emitting radionuclide which is gadolinium-159 (Gd-159), holmium-166 (Ho-166), lutetium-177 (Lu-177), samarium-153 (Sm-153) or ytterbium-175 (Yb-175) with an aminophosphonic acid derivative which is ethylenediaminetetramethylenephosphonic acid (EDTMP), diethylenetriaminepentamethylenephosphonic acid (DTPMP), hydroxyethylethylenediaminetrimethylenephosphonic acid (HEEDTMP), nitrilotrimethylenephosphonic acid (NTMP) or tris(2-aminoethyl) aminehexamethylenephosphonic acid (TTHMP) or a physiologically acceptable salt of such a complex.

2. A complex as claimed in Claim 1, characterized in that said radionuclide is Gd-159, Lu-177, Sm-153 or Yb-175.

3. A complex as claimed in Claim 2, characterized in that said radionuclide is Lu-177, Sm-153 or Yb-175.

4. A complex as claimed in Claim 3, characterized in that said radionuclide is Sm-153.

5. A complex as claimed in Claim 3, characterized in that said radionuclide is Lu-177.

6. A complex as claimed in Claim 3, characterized in that said radionuclide is Yb-175.

7. A complex as claimed in Claim 2, characterized in said radionuclide is Gd-159.

8. A complex as claimed in any one of Claims 1 to 7, characterized in that said aminophosphonic acid derivative is ethylenediaminetetramethylenephosphonic acid (EDTMP).

9. A complex as claimed in any one of Claims 1 to 7, characterized in that said aminophosphonic acid derivative is diethylenetriaminepentamethylenephosphonic acid (DTPMP).

10. A complex as claimed in any one of Claims 1 to 7, characterized in that said aminophosphonic acid derivative is hydroxyethylethylenediaminetrimethylene phosphonic acid (HEEDTMP).

11. A complex as claimed in any one of Claims 1 to 7, characterized in that said aminophosphonic acid derivative is nitrilotrimethylenephosphonic acid (NTMP).

12. A complex as claimed in any one of Claims 1 to 7, characterized in that said aminophosphonic acid derivative is tris(2-aminoethyl) aminehexamethylenephosphonic acid (TTHMP).

13. A complex as claimed in Claim 1 wherein the radionuclide is Sm-153, and the aminophosphonic acid derivative is ethylenediaminetetramethylenephosphonic acid (EDTMP).

14. A complex as claimed in any one of the preceding Claims, wherein the molar ratio of the aminophosphonic acid derivative to the metal is from 0.1:1 to 3000:1

15. A complex as claimed in Claim 14, wherein the molar ratio of the aminophosphonic acid derivative to the metal is from 1:1 to 2000:1.

16. A complex as claimed in Claim 15, wherein the molar ratio of the aminophosphonic acid derivative to the metal is from 1:1 to 1000:1.

17. A process for the preparation of a therapeutically effective complex as claimed in Claim 1, characterized in that a radionuclide which is Gd-159, Ho-166, Lu-177, Sm-153, or Yb-175 is contacted with an aminophosphonic acid derivative which is EDTMP, DTPMP, HEEDTMP, NTMP or TTHMP.

18. A process as claimed in Claim 17, wherein the said contact is carried out in the presence of water at a pH of from 5 to 11.

19. A process as claimed in Claim 17 or Claim 18, wherein the said contact is carried out at an elevated

15

temperature, preferably of from 60° -70° C.

20. A process as claimed in any one of Claims 17 to 19, including the step of adjusting the pH of the complex to from 7 to 8.

21. A process as claimed in any one of Claims 17 to 20, characterized in that said radionuclide is Gd-159, Lu-177, Sm-153 or Yb-175.

22. A process as claimed in Claim 21, characterized in that said radionuclide is Lu-177, Sm-153 or Yb-175.

23. A process as claimed in Claim 22, characterized in that said radionuclide is Sm-153.

24. A process as claimed in Claim 22, characterized in that said radionuclide is Lu-177.

25. A process as claimed in Claim 22, characterized in that said radionuclide is Yb-175.

26. A process as claimed in Claim 22, charaterized in that said radionuclide is Gd-159.

27. A process as claimed in any one of Claims 17 to 26, characterized in that said aminophosphonic acid derivative is EDTMP.

28. A process as claimed in Claim 21, wherein the radionuclide is Sm-153, and the aminophosphonic acid derivative is ethylenediaminetetramethylenephosphonic acid (EDTMP).

29. A process as claimed in any one of Claims 17 to 26, characterized in that said aminophosphonic acid derivative is DTPMP.

30. A process as claimed in any one of Claims 17 to 26, characterized in that said aminophosphonic acid derivative is HEEDTMP.

31. A process as claimed in any one of Claims 17 to 26, characterized in that said aminophosphonic acid derivative is NTMP.

32. A process as claimed in any one of Claims 17 to 26, characterized in that said aminophosphonic acid derivative is TTHMP.

33. A process as claimed in any one of Claims 17 to 32, characterized in that said aminophosphonic acid derivative and said radionuclide are contacted in a molar ratio of from 0.1:1 to 3000:1.

34. A complex as claimed in any one of Claims 1 to 16, for use in bone cancer therapy or for use in the palliation of pain associated with bone cancer.

35. The use of a complex as claimed in any one of Claims 1 to 16, in the manufacture of a medicament for the treatment of cancer or for the palliation of pain associated with cancer.

36. A pharmaceutical composition comprising a complex as claimed in any one of Claims 1 to 16 together with a pharmaceutical acceptable diluent.

**Revendications**

1. Complexe thérapeutiquement actif d'un radionuclide émetteur de particules qui est le gadolinium-159 (Gd-159), l'holmium-166 (Ho-166), le lutétium-177 (Lu-177), le samarium-153 (Sm-153) ou l'ytterbium-175 (Yb-175) avec un dérivé d'acide aminophosphonique qui est l'acide éthylènediaminetétraméthylènephosphonique (EDTMP), l'acide diéthylènetriaminepentaméthylènephosphonique (DTPMP), l'acide hydroxyéthyléthylènediaminetriméthylènephosphonique (HEEDTMP), l'acide nitrilotriméthylènephosphonique (NTMP) ou l'acide tris-(2-aminoéthyl)aminehexaméthylènephosphonique (TTHMP) ou un sel physiologiquement acceptable d'un tel complexe.

2. Complexe selon la revendication 1, **caractérisé** en ce que ledit radionuclide est Gd-159, Lu-177, Sm-153 ou Yb-175.

3. Complexe selon la revendication 2, **caractérisé** en ce que ledit radionuclide est Lu-177, Sm-153 ou Yb-175.

4. Complexe selon la revendication 3, **caractérisé** en ce que ledit radionuclide est Sm-153.

5. Complexe selon la revendication 3, **caractérisé** en ce que ledit radionuclide est Lu-177.

6. Complexe selon la revendication 3, **caractérisé** en ce que ledit radionuclide est Yb-175.

7. Complexe selon la revendication 2, **caractérisé** en ce que ledit radionuclide est Gd-159.

8. Complexe selon l'une des revendications 1 à 7, **caractérisé** en ce que ledit dérivé d'acide aminophosphonique est l'acide éthylènediaminetétraméthylènephosphonique (EDTMP).

9. Complexe selon l'une quelconque des revendications 1 à 7, **caractérisé** en ce que ledit dérivé d'acide aminophosphonique est l'acide diéthylènetriaminepentaméthylènephosphonique (DTPMP).

10. Complexe selon l'une quelconque des revendications 1 à 7, **caractérisé** en ce que ledit dérivé d'acide aminophosphonique est l'acide hydroxyéthyléthylènediaminetriméthylènephosphonique (HEEDTMP).

11. Complexe selon l'une quelconque des revendications 1 à 7, **caractérisé** en ce que ledit dérivé d'acide aminophosphonique est l'acide nitrilotriméthylènephosphonique (NTMP).

12. Complexe selon l'une quelconque des revendications 1 à 7, **caractérisé** en ce que ledit dérivé d'acide aminophosphonique est l'acide tris-(2-aminoéthyl)aminehexaméthylènephosphonique (TTHMP).

13. Complexe selon la revendication 1, dans lequel le radionuclide est Sm-153 et le dérivé d'acide aminophosphonique est l'acide éthylènediaminetétraméthylènephosphonique (EDTMP).

14. Complexe selon l'une quelconque des revendications précédentes, dans lequel le rapport molaire du dérivé d'acide aminophosphonique au métal est de 0,1:1 à 3000:1.

15. Complexe selon la revendication 14, dans lequel le rapport molaire du dérivé d'acide aminophosphonique au métal est de 1:1 à 2000:1.

16. Complexe selon la revendication 15, dans lequel le rapport molaire du dérivé d'acide aminophosphonique au métal est de 1:1 à 1000:1.

17. Procédé de préparation d'un complexe thérapeutiquement actif tel que revendiqué dans la revendication 1, **caractérisé** en ce que l'on met en contact un radionuclide qui est Gd-159, Ho-166, Lu-177, Sm-153 ou Yb-175 avec un dérivé d'acide aminophosphonique qui est EDTMP, DTPMP, HEEDTMP, NTMP ou TTHMP.

18. Procédé selon la revendication 17, dans lequel ledit contact est effectué en présence d'eau à un pH de 5 à 11.

19. Procédé selon la revendication 17 ou 18, dans lequel ledit contact est effectué à une température élevée, de préférence de 60° à 70° C.

20. Procédé selon l'une quelconque des revendications 17 à 19, comprenant l'étape d'ajustement du pH du complexe à une valeur de 7 à 8.

21. Procédé selon l'une quelconque des revendications 17 à 20, **caractérisé** en ce que ledit radionuclide est Gd-159, Lu-177, Sm-153 ou Yb-175.

**22.** Procédé selon la revendication 21, **caractérisé** en ce que ledit radionuclide est Lu-177, Sm-153 ou Yb-175.

**23.** Procédé selon la revendication 22, **caractérisé** en ce que ledit radionuclide est Sm-153.

**24.** Procédé selon la revendication 22, **caractérisé** en ce que ledit radionuclide est Lu-177.

**25.** Procédé selon la revendication 22, **caractérisé** en ce que ledit radionuclide est Yb-175.

**26.** Procédé selon la revendication 22, **caractérisé** en ce que ledit radionuclide est Gd-159.

**27.** Procédé selon l'une quelconque des revendications 17 à 26, **caractérisé** en ce que ledit dérivé d'acide aminophosphonique est EDTMP.

**28.** Procédé selon la revendication 21, dans lequel le radionuclide est Sm-153 et le dérivé d'acide aminophosphonique est l'acide éthylènediaminetétraméthylènephosphonique (EDTMP).

**29.** Procédé selon l'une quelconque des revendications 17 à 26, **caractérisé** en ce que ledit dérivé d'acide aminophosphonique est DTPMP.

**30.** Procédé selon l'une quelconque des revendications 17 à 26, **caractérisé** en ce que ledit dérivé d'acide aminophosphonique est HEEDTMP.

**31.** Procédé selon l'une quelconque des revendications 17 à 26, **caractérisé** en ce que ledit dérivé d'acide aminophosphonique est NTMP.

**32.** Procédé selon l'une quelconque des revendications 17 à 26, **caractérisé** en ce que ledit dérivé d'acide aminophosphonique est TTHMP.

**33.** Procédé selon l'une quelconque des revendications 17 à 26, **caractérisé** en ce que ledit dérivé d'acide aminophosphonique et ledit radionuclide sont mis en contact dans un rapport molaire de 0,1:1 à 3000:1.

**34.** Complexe tel que revendiqué dans l'une quelconque des revendications 1 à 16, pour utilisation dans le traitement du cancer des os ou pour utilisation dans le soulagement de la douleur accompagnant le cancer des os.

**35.** Utilisation d'un complexe tel que revendiqué dans l'une quelconque des revendications 1 à 16 dans la fabrication d'un médicament pour le traitement du cancer ou pour le soulagement de la douleur accompagnant le cancer.

**36.** Composition pharmaceutique comprenant un complexe tel que revendiqué dans l'une quelconque des revendications 1 à 16 et un diluant pharmaceutiquement acceptable.

**Ansprüche**

**1.** Therapeutisch wirksamer Komplex eines Teilchenemittierenden Radionuklids, das Gadolinium-159 (Gd-159), Holmium-166 (Ho-166), Lutetium-177 (Lu-177), Samarium-153 (Sm-153) oder Ytterbium-175 (Yb-175) ist, mit einem Aminophosphonsäure-Derivat, das Ethylendiamintetramethylenphosphonsäure (EDTMP), Diethylentriaminpentamethylenphosphonsäure (DTPMP), Hydoxyethylethylendiamintrimethylenphosphonsäure (HEEDTMP), Nitriltrimethylenphosphonsäure (NTMP) oder Tris(2-aminoethyl)-aminhexamethylenphosphonsäure (TTHMP) ist, oder ein physiologisch verträgliches Salz eines derartigen Komplexes.

**2.** Komplex nach Anspruch 1, dadurch gekennzeichnet, daß das Radionuklid Gd-159, Lu-177, Sm-153 oder Yb-175 ist.

**3.** Komplex nach Anspruch 2, dadurch gekennzeichnet, daß das Radionuklid Lu-177, Sm-153 oder Yb-175 ist.

4. Komplex nach Anspruch 3, dadurch gekennzeichnet, daß das Radionuklid Sm-153 ist.

5. Komplex nach Anspruch 3, dadurch gekennzeichnet, daß das Radionuklid Lu-177 ist.

6. Komplex nach Anspruch 3, dadurch gekennzeichnet, daß das Radionuklid Yb-175 ist.

7. Komplex nach Anspruch 2, dadurch gekennzeichnet, daß das Radionuklid Gd-159 ist.

8. Komplex nach einem der Ansprüche 1 bis 7, dadurch gekennzeichnet, daß das Aminophosphonsäure-Derivat Ethylendiamintetramethylenphosphonsäure (EDTMP) ist.

9. Komplex nach einem der Ansprüche 1 bis 7, dadurch gekennzeichnet, daß das Aminophosphonsäure-Derivat Diethylentriaminpentamethylenphosphonsäure (DTPMP) ist.

10. Komplex nach einem der Ansprüche 1 bis 7, dadurch gekennzeichnet, daß das Aminophosphonsäure-Derivat Hydroxyethylethylendiamintrimethylenphosphonsäure (HEEDTMP) ist.

11. Komplex nach einem der Ansprüche 1 bis 7, dadurch gekennzeichnet, daß das Aminophosphonsäure-Derivat Nitriltrimethylenphosphonsäure (NTMP) ist.

12. Komplex nach einem der Ansprüche 1 bis 7, dadurch gekennzeichnet, daß das Aminophosphonsäure-Derivat Tris(2-aminoethyl)aminhexamethylenphosphonsäure (TTHMP) ist.

13. Komplex nach Anspruch 1, worin das Radionuklid Sm-153 ist und das Aminophosphonsäure-Derivat Ethylendiamintetramethylenphosphonsäure (EDTMP) ist.

14. Komplex nach einem der vorhergehenden Ansprüche, worin das molare Verhältnis des Aminophosphonsäure-Derivats zu dem Metall von 0,1:1 bis 3000:1 ist.

15. Komplex nach Anspruch 14, worin das molare Verhältnis des Aminophosphonsäure-Derivats zu dem Metall von 1:1 bis 2000:1 ist.

16. Komplex nach Anspruch 15, worin das molare Verhältnis des Aminophosphonsäure-Derivats zu dem Metall von 1:1 bis 1000:1 ist.

17. Verfahren zur Herstellung eines therapeutisch wirksamen Komplexes nach Anspruch 1, dadurch gekennzeichnet, daß ein Radionuklid, das Gd-159, Ho-166, Lu-177, Sm-153 oder Yb-175 ist, mit einem Aminophoshonsäure-Derivat kontaktiert wird, das EDTMP, DTPMP, HEEDTMP, NTMP oder TTHMP ist.

18. Verfahren nach Anspruch 17, worin der Kontakt in Gegenwart von Wasser bei einem pH von 5 bis 11 durchgeführt wird.

19. Verfahren nach Anspruch 17 oder 18, worin der Kontakt bei einer erhöhten Temperatur, vorzugsweise von 60°-70° C durchgeführt wird.

20. Verfahren nach einem der Ansprüche 17 bis 19, das den Schritt der pH-Einstellung des Komplexes auf 7 bis 8 beinhaltet.

21. Verfahren nach einem der Ansprüche 17 bis 20, dadurch gekennzeichnet, daß das Radionuklid Gd-159, Lu-177, Sm-153 oder Yb-175 ist.

22. Verfahren nach Anspruch 21, dadurch gekennzeichnet, daß das Radionuklid Lu-177, Sm-153 oder Yb-175 ist.

23. Verfahren nach Anspruch 22, dadurch gekennzeichnet, daß das Radionuklid Sm-153 ist.

**24.** Verfahren nach Anspruch 22, dadurch gekennzeichnet, daß das Radionuklid Lu-177 ist.

**25.** Verfahren nach Anspruch 22, dadurch gekennzeichnet, daß das Radionuklid Yb-175 ist.

**26.** Verfahren nach Anspruch 21, dadurch gekennzeichnet, daß das Radionuklid Gd-159 ist.

**27.** Verfahren nach einem der Ansprüche 17 bis 26, dadurch gekennzeichnet, daß das Aminophosphonsäure-Derivat EDTMP ist.

**28.** Verfahren nach Anspruch 21, worin das Radionuklid Sm-153 und das Aminophosphonsäure-Derivat Ethylendiamintetramethylenphosphonsäure (EDTMP) ist.

**29.** Verfahren nach einem der Ansprüche 17 bis 26, dadurch gekennzeichnet, daß das Aminophosphonsäure-Derivat DTPMP ist.

**30.** Verfahren nach einem der Ansprüche 17 bis 26, dadurch gekennzeichnet, daß das Aminophosphonsäure-Derivat HEEDTMP ist.

**31.** Verfahren nach einem der Ansprüche 17 bis 26, dadurch gekennzeichnet, daß das Aminophosphonsäure-Derivat NTMP ist.

**32.** Verfahren nach einem der Ansprüche 17 bis 26, dadurch gekennzeichnet, daß das Aminophosphonsäure-Derivat TTHMP ist.

**33.** Verfahren nach einem der Ansprüche 17 bis 32, dadurch gekennzeichnet, daß das Aminophosphonsäure-Derivat und das Radionuklid in einem molaren Verhältnis von 0,1:1 bis 3000:1 kontaktiert werden.

**34.** Komplex nach einem der Ansprüche 1 bis 16 zur Verwendung bei der Therapie von Knochenkrebs oder zur Verwendung bei der Linderung der mit Knochenkrebs assoziierten Schmerzen.

**35.** Verwendung eines Komplexes nach einem der Ansprüche 1 bis 16 bei der Herstellung eines Medikaments zur Behandlung von Krebs oder zur Linderung der mit Krebs assoziierten Schmerzen.

**36.** Pharmazeutische Zusammensetzung, die einen Komplex nach einem der Ansprüche 1 bis 16 zusammen mit einem pharmazeutisch verträglichen Verdünnungsmittel enthält.